(19) Europäisches Patentamt
European Patent Office
Office européen des brevets

(11) Numéro de publication : **0 530 058 A1**

(12) # DEMANDE DE BREVET EUROPEEN

(21) Numéro de dépôt : **92401849.2**

(22) Date de dépôt : **30.06.92**

(51) Int. Cl.[5] : **C07D 513/04,** A61K 31/54, // (C07D513/04, 285:00, 277:00)

(30) Priorité : **04.07.91 FR 9108354**

(43) Date de publication de la demande : **03.03.93 Bulletin 93/09**

(84) Etats contractants désignés : **PT**

(71) Demandeur : **RHONE-POULENC RORER SA
20, avenue Raymond Aron
F-92160 Antony (FR)**

(72) Inventeur : **Audiau, François
9 rue Guérin
F-94220 Charenton Le Pont (FR)**
Inventeur : **Jimonet, Patrick
13, Avenue de Normandie
F-78450 Villepreux (FR)**
Inventeur : **Mignani, Serge
129 Avenue du Général Leclerc
F-75014 Paris (FR)**

(74) Mandataire : **Pilard, Jacques et al
RHONE-POULENC RORER S.A. Direction
Brevets (t144) 20 avenue Raymond Aron
F-92165 Antony Cédex (FR)**

(54) **Dérivés de 1,2,4-thiadiazino(3,4-b)benzothiazole, leur préparation et médicaments les contenant.**

(57) Composés de formule :

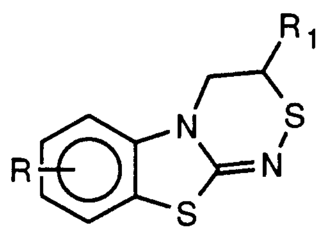

(I)

dans laquelle R représente un radical polyfluoroalcoxy, polyfluoroalkyle, alcoxy ou alkyle et $R_1$ représente un atome d'hydrogène ou un radical alkyle, leurs sels, leur préparation et les médicaments les contenant.

EP 0 530 058 A1

La présente invention concerne des dérivés de formule:

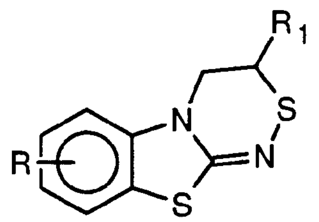

(I)

leurs sels, leur préparation et les médicaments les contenant.

Dans la formule (I),

- R représente un radical polyfluoroalcoxy, polyfluoroalkyle, alcoxy ou alkyle,
- $R_1$ représente un atome d'hydrogène ou un radical alkyle.

Dans les définitions qui précédent et celles qui seront citées ci-après, les radicaux et les portions alkyle et alcoxy contiennent 1 à 4 atomes de carbone en chaîne droite ou ramifiée.

Les radicaux polyfluoroalkyle sont de préférence les radicaux trifluorométhyle, trifluoro-2,2,2 éthyle et pentafluoroéthyle.

Les radicaux polyfluoroalcoxy sont de préférence les radicaux trifluorométhoxy, pentafluoroéthoxy, trifluoro-2,2,2 éthoxy et tétrafluoro-1,1,2,2 éthoxy.

L'invention concerne également les sels d'addition des composés de formule (I) avec les acides minéraux ou organiques.

Les composés de formule (I) peuvent être préparés par cyclisation d'un dérivé de formule :

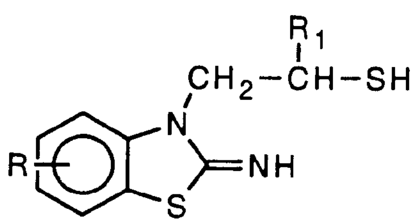

(II)

dans laquelle R et $R_1$ ont les mêmes significations que dans la formule (I).

Cette cyclisation s'effectue généralement en milieu oxydant (brome,chlore, iode, triiodure de potassium, eau oxygénée, chloramine T par exemple) au sein d'un solvant inerte tel qu'un alcool (méthanol, éthanol par exemple), un solvant chloré (chloroforme, chlorure de méthylène par exemple), un solvant aromatique (benzène, toluène par exemple), une cétone (acétone par exemple), à une température variant de 20 à 50°C.

Les dérivés de formule (II) peuvent être obtenus par hydrolyse d'un dérivé de formule :

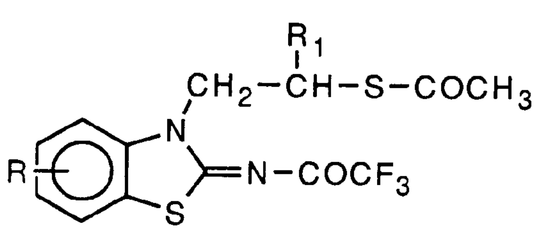

(III)

dans laquelle R et $R_1$ ont les mêmes significations que dans la formule (I).

Cette hydrolyse s'effectue de préférence au moyen d'une base telle qu'un carbonate de métal alcalin (sodium, potassium par exemple), un hydroxyde de métal alcalin (soude, potasse par exemple), au sein d'un solvant inerte tel qu'un alcool, une cétone, l'eau ou un mélange de ces solvants, à une température comprise entre 20 et 50°C.

Les dérivés de formule (III) peuvent être obtenus par action d'acide thioacétique sur un dérivé de formule:

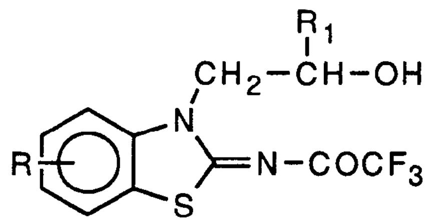

(IV)

dans laquelle R et $R_1$ ont les mêmes significations que dans la formule (I).

Cette réaction s'effectue de préférence au sein d'un solvant inerte tel que le tétrahydrofuranne, au moyen de triphénylphosphine et d'azodicarboxylate d'éthyle, à une température comprise entre 0 et 25°C.

Les dérivés de formule (IV) peuvent être préparés par application ou adaptation de la méthode décrite dans le brevet EP409692.

Les mélanges réactionnels obtenus par les divers procédés décrits précédemment sont traités suivant des méthodes classiques physiques (évaporation, extraction, distillation, chromatographie, cristallisation....) ou chimiques (formation de sels....).

Les composés de formule (I) sous forme de base libre peuvent être éventuellement transformés en sels d'addition avec un acide minéral ou organique, par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés interfèrent avec la transmission glutamatergique et sont donc utiles dans le traitement et la prévention des phénomènes liés au glutamate. Ces phénomènes sont notamment les manifestations épileptogènes et/ou convulsives, les troubles schizophréniques et en particulier les formes déficitaires de la schizophrénie, les troubles du sommeil, l'anxiété, les phénomènes liés à l'ischémie cérébrale ainsi que les troubles neurologiques liés au vieillissement où le glutamate peut être impliqué telles que la maladie d'Alzheimer, la maladie d'Huntington, la sclérose latérale amyotrophique et l'atrophie olivopontocérébelleuse.

L'activité de ces produits comme antiglutamate a été déterminée sur les convulsions induites par le glutamate selon une technique inspirée de celle de I. P. LAPIN, J. Neural. Transmission, 54, 229-238 (1982); l'injection du glutamate par voie intracérébroventriculaire étant effectuée selon une technique inspirée de celle de R. CHERMAT et P. SIMON, J. Pharmacol. (Paris), 6, 489-492 (1975). Leur $DE_{50}$ est inférieure ou égale à 10 mg/kg.

Les composés de formule (I) présentent une toxicité faible. Leur $DL_{50}$ est supérieure à 15 mg/kg par voie IP chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

L'exemple suivant, donné à titre non limitatif, montre comment l'invention peut être mise en pratique.

**EXEMPLE 1**

A 1,2 g de trihydrate de chloramine T (Prolabo) en solution dans 15 $cm^3$ de méthanol, on ajoute progressivement 1,2 g de 2-imino-3-(2-mercaptoéthyl)-6-trifluorométhoxy-benzothiazoline à une température voisine de 20°C. La réaction est poursuivie 2 heures à cette température. Le méthanol est évaporé sous pression réduite et le résidu blanchâtre repris dans 50 $cm^3$ d'eau distillée. La phase organique est extraite par 2 fois 30 $cm^3$ d'éther éthylique, lavée à l'eau distillée puis séchée sur sulfate de magnésium. Après filtration et concentration à sec sous pression réduite, on obtient 1,2 g d'une mousse blanche purifiée par flash-chromatographie sur silice avec un mélange acétate d'éthyle-cyclohexane (40-60 en volumes) comme éluant. 0,9 g de 3,4-dihydro-8-trifluorométhoxy-1,2,4-thiadiazino[3,4-b]benzothiazole sont obtenus sous forme d'une huile incolore qui par addition d'acide oxalique dans l'acétone, donne 0,8g d'oxalate fondant à 178°C.

La 2-imino-3-(2-mercaptoéthyl)-6-trifluorométhoxy-benzothiazoline peut être préparée de la façon suivante : à 15,1 g de 3-(2-acétylthioéthyl)-2-trifluoroacétylimino-6-trifluorométhoxy-benzothiazoline en solution dans 500 $cm^3$ de méthanol, on ajoute 75 $cm^3$ d'une solution de carbonate de potassium à 7% dans l'eau à une température voisine de 20°C. Après 72 heures de réaction à cette température, le méthanol est évaporé sous pression réduite et le résidu solide repris dans 500 $cm^3$ d'eau distillée. L'insoluble blanc est filtré, lavé à l'eau

puis séché. Après purification par flash-chromatographie sur colonne de silice avec de l'acétate d'éthyle comme éluant, on obtient 6,3 g de 2-imino-3-(2-mercaptoéthyl)-6-trifluorométhoxy-benzothiazoline sous forme de solide blanc fondant à 110°C.

La 3-(2-acétylthioéthyl)-2-trifluoroacétylimino-6-trifluorométhoxy-benzothiazoline peut être obtenue de la manière suivante : dans un ballon placé sous azote, on introduit 21 g de triphénylphosphine et 150 $cm^3$ de tétrahydrofurane. A ce mélange agité à 0-5°C, on ajoute, goutte à goutte, en environ 30 minutes, une solution de 12,8 $cm^3$ d'azodicarboxylate d'éthyle dans 100 $cm^3$ de tétrahydrofurane. L'agitation est poursuivie 1 heure à cette température. Une solution de 14,2 g de 3-(2-hydroxyéthyl)-2-trifluoroacétylimino-6-trifluorométhoxy-benzothiazoline et de 5,6 $cm^3$ d'acide thioacétique dans 250 $cm^3$ de tétrahydrofurane est alors ajoutée goutte à goutte en environ 30 minutes à 0°C. Cette température est maintenue 1 heure puis la réaction poursuivie 15 heures à une température voisine de 20°C. Après concentration à sec sous pression réduite, le résidu est purifié par flash-chromatographie sur colonne de silice avec un mélange cyclohexane-acétate d'éthyle (95-5 en volumes) comme éluant. On obtient ainsi 15,3 g de produit attendu sous forme d'une poudre blanche fondant à 120°C.

La 3-(2-hydroxyéthyl)-2-trifluoroacétylimino-6-trifluorométhoxy-benzothiazoline peut être préparée selon la méthode décrite dans le brevet EP 409692.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, intraveineuse, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention interfèrent avec la transmission glutamatergique et sont donc particulièrement utiles dans le traitement et la prévention des désordres liés au glutamate. Ces composés sont notamment utiles pour le traitement ou la prévention des manifestations épileptogènes et/ou convulsives, des troubles schizophréniques et notamment des formes déficitaires de la schizophrénie, des troubles du sommeil, de l'anxiété, des phénomènes liés à l'ischémie cérébrale ainsi que des troubles neurologiques liés au vieillissement où le glutamate peut être impliqué telles que la maladie d'Alzheimer, la maladie d'Huntington, la sclérose latérale amyotrophique et l'atrophie olivopontocérébelleuse.

Les doses dépendent de l'effet recherché, de la durée du traitement et de la voie d'administration utilisée; elles sont généralement comprises entre 30 et 300 mg par jour par voie orale pour un adulte avec des doses unitaires allant de 10 mg à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

- 3,4-dihydro-8-trifluorométhoxy-1,2,4-thiadiazino[3,4-b] benzothiazole .......... 50 mg
- Cellulose .......... 18 mg
- Lactose .......... 55 mg
- Silice colloïdale .......... 1 mg
- Carboxyméthylamidon sodique .......... 10 mg
- Talc .......... 10 mg
- Stéarate de magnésium .......... 1 mg

EXEMPLE B

On prépare selon la technique habituelle de comprimés dosés à 50 mg de produit actif ayant la composition suivante:

- 3,4-dihydro-8-trifluorométhoxy-1,2,4-thiadiazino[3,4-b] benzothiazole .......... 50 mg
- Lactose .......... 104 mg
- Cellulose .......... 40 mg
- Polyvidone .......... 10 mg
- Carboxyméthylamidon sodique .......... 22 mg
- Talc .......... 10 mg
- Stéarate de magnésium .......... 2 mg
- Silice colloïdale .......... 2 mg
- Mélange d'hydroxyméthylcellulose, glycérine, oxyde de titane (72-3,5-24,5) q.s.p. 1 comprimé pelliculé terminé à 245 mg

EXEMPLE C

On prépare une solution injectable contenant 10 mg de produit actif ayant la composition suivante :

- 3,4-dihydro-8-trifluorométhoxy-1,2,4-thiadiazino[3,4-b] benzothiazole .......... 10 mg
- Acide benzoïque .......... 80 mg
- Alcool benzylique .......... 0,06 cm3
- Benzoate de sodium .......... 80 mg
- Ethanol à 95 % .......... 0,4 cm3
- Hydroxyde de sodium .......... 24 mg
- Propylène glycol .......... 1,6 cm3
- Eau .......... q.s.p. 4 cm3

**Revendications**

**1 -** Composés de formule:

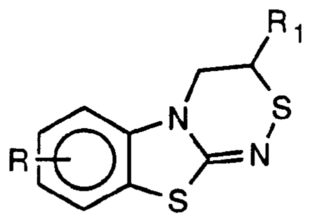

(I)

dans laquelle R représente un radical polyfluoroalcoxy, polyfluoroalkyle, alcoxy ou alkyle et $R_1$ représente un atome d'hydrogène ou un radical alkyle et leurs sels avec un acide minéral ou organique, étant entendu que les radicaux et portions alkyle et les portions alcoxy contiennent 1 à 4 atomes de carbone en chaîne droite ou ramifiée.

**2 -** Composés de formule (I) selon la revendication 1 pour lesquels R représente un radical trifluorométhyle, trifluoro-2,2,2 éthyle, trifluorométhoxy, pentafluoroéthyle, trifluoro-2,2,2 éthoxy ou tétrafluoro-1,1,2,2 éthoxy.

**3 -** Le 3,4-dihydro-8-trifluorométhoxy-1,2,4-thiadiazino[3,4-b]benzothiazole et ses sels avec un acide minéral ou organique.

**4 -** Procédé de préparation des composés de formule (I) selon la revendication 1 caractérisé en ce que l'on cyclise un dérivé de formule :

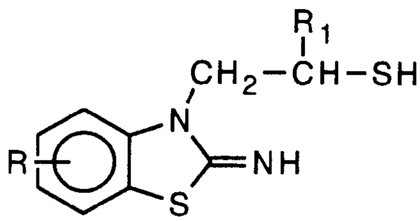

(II)

dans laquelle R et $R_1$ ont les mêmes significations que dans la revendication 1.

**5 -** Médicaments caractérisés en ce qu'ils contiennent en tant que principe actif au moins un composé de formule (I) selon la revendication 1 ou un sel d'un tel composé.

**6 -** Médicaments contenant en tant que principe actif le 3,4-dihydro-8-trifluorométhoxy-1,2,4-thiadiazino[3,4-b]benzothiazole ou un sel de ce composé.

**7 -** Médicaments selon l'une des revendications 5 et 6 qui interfèrent avec la transmission glutamatergique.

Office européen des brevets

# RAPPORT DE RECHERCHE EUROPEENNE

Numero de la demande

EP 92 40 1849

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | EP-A-0 409 692 (RHONE-POULENC SANTE)<br>* revendications 1,2,5-7; page 3, formule (VI); page 4, lignes 49-55 *<br>--- | 1,4,5,7 | C07D513/04<br>A61K31/54<br>//(C07D513/04, 285:00,277:00) |
| A | EP-A-0 375 510 (RHONE-POULENC SANTE)<br>* revendications 1,2,7,8; page 4, formule (VII); page 5, lignes 21-27 *<br>--- | 1,4,5,7 | |
| A | EP-A-0 374 040 (RHONE-POULENC SANTE)<br>* revendications 1,7,8; page 5, formule (VI) et lignes 31-37 *<br>--- | 1,4,5,7 | |
| A | JOURNAL OF ORGANIC CHEMISTRY.<br>vol. 44, no. 22, 1979, EASTON US<br>pages 3847 - 3858;<br>J.J. KRUTAK ET AL.: 'Chemistry of Ethenesulfonyl Fluoride. Fluorosulfonylethylation of Organic Compounds'<br>* page 3852, tableau IV, composé no. 46 *<br>--- | 1 | |
| A | CHEMISCHE BERICHTE.<br>vol. 100, 1967, WEINHEIM DE<br>pages 2159 - 2163;<br>H. BEECKEN: 'Über die Cycloaddition heterocyclischer N-Sulfinyl-amine an Bicyclo[2.2.1]hepten und Äthoxyacetylen'<br>* page 2160, composé 4 *<br>----- | 1 | DOMAINES TECHNIQUES RECHERCHES (Int. Cl.5 )<br>C07D |

Le présent rapport a été établi pour toutes les revendications

| Lieu de la recherche | Date d'achèvement de la recherche | Examinateur |
|---|---|---|
| BERLIN | 06 OCTOBRE 1992 | CHRISTIAN HASS |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)